# EUROPÄISCHE PATENTSCHRIFT

(11) **EP 1 778 113 B1**
(45) Veröffentlichungstag und Bekanntmachung des Hinweises auf die Patenterteilung: **12.09.2012**
(21) Anmeldenummer: 05767564.7
(22) Anmeldetag: 14.07.2005
(51) Int. Cl.: A61B 18/14

(54) **ELEKTROCHIRURGISCHES INSTRUMENT**
ELECTROSURGICAL INSTRUMENT
INSTRUMENT ELECTROCHIRUGICAL

(30) Priorität: 11.08.2004 DE 102004039051; 18.11.2004 DE 102004055670
(43) Veröffentlichungstag der Anmeldung: 02.05.2007
(73) Patentinhaber: ERBE Elektromedizin GmbH, 72072 Tübingen (DE)
(72) Erfinder: HAFNER, Dieter, 72072 Tübingen (DE)
(74) Vertreter: Bohnenberger, Johannes
(86) Internationale Anmeldenummer: PCT/EP2005/007681
(87) Internationale Veröffentlichungsnummer: WO 2006/018083

(56) Entgegenhaltungen:
- US-A- 5 458 598
- US-A1- 2001 037 109
- US-A1- 2004 049 185
- US-B1- 6 267 761

## Beschreibung

Die Erfindung betrifft ein elektrochirurgisches Instrument nach dem Oberbegriff des Patentanspruches 1.

Elektrochirurgische Instrumente werden seit vielen Jahren in der Hochfrequenz-Chirurgie eingesetzt, um biologisches Gewebe insbesondere zu koagulieren, aber auch zu schneiden. Bei einer Koagulation wird ein hochfrequenter Strom durch das zu behandelnde Gewebe geleitet, so dass sich dieses aufgrund Eiweißkoagulation und Dehydratation verändert. Das Gewebe zieht sich dabei derart zusammen, dass die Gefäße verschlossen und Blutungen gestillt werden. Nach erfolgter Koagulation ist das Gewebe beispielsweise mittels eines mechanisch arbeitenden Schneidinstruments durchtrennbar.

Elektrochirurgische Vorgänge sind sowohl monopolar als auch bipolar durchführbar. Bei der monopolaren Technik weist das elektrochirurgische Instrument nur eine einzige Stromzuführung auf, das zu behandelnde Gewebe (bzw. ein Patient) ist demgemäß auf das andere Potential zu legen. Immer mehr an Bedeutung gewinnen jedoch bipolare Instrumente, die mit zwei voneinander elektrisch isolierten Abschnitten ausgebildet sind. Der Stromweg zwischen den Elektrodenteilen ist damit kalkulierbar und verläuft nicht weite Strecken durch den Körper des Patienten. Damit reduziert sich die Beeinflussung von beispielsweise Herzschrittmachern oder sonstigen Geräten, die während der Operation an dem Patienten angeschlossen sind.

Bipolare Koagulationsinstrumente weisen im Wesentlichen zwei gelenkig miteinander verbundene Branchen auf, an deren proximalen Enden Griffeinrichtungen zur Handhabung der Branchen vorgesehen sind. An distalen Enden der Branchen befinden sich Elektrodenteile mit Koagulationsflächen zum Fassen von Gewebe und zum Durchleiten des Koagulationsstromes durch das Gewebe. Der von einem HF-Generator gelieferte HF-Strom wird dazu über Stromzuführungseinrichtungen zu den Elektrodenteilen des bipolaren Instruments geleitet.

Nach erfolgter Koagulation wird im Allgemeinen der Schneidprozess mittels eines Schneidinstruments durchgeführt. Bei einem mechanischen Schneiden ist von dem Chirurgen Kraft aufzuwenden, um den Schnitt durchführen zu können, was einerseits eine mechanische Belastung des Schneidinstruments bewirkt und andererseits einen abrasiven Verschleiß der Schneidabschnitte begünstigt. Verschleißbedingt bleiben zudem Partikel der Schneidabschnitte in dem behandelten Gewebe zurück, so dass hier eine erhöhte Infektionsgefahr besteht.

Eine Reduzierung der aufzuwendenden Schneidkraft wird bei herkömmlichen Schneidinstrumenten beispielsweise durch aufwändig herzustellende und teure Übersetzungsmechanismen bewirkt. Gleichzeitig sind die Instrumente häufig äußerst stabil ausgelegt, um der mechanischen Belastung zu begegnen. Instrumente mit großem Bauraum sind jedoch gerade für endoskopische Verfahren ungeeignet.

Andere Lösungen sehen vor, die Schneidabschnitte zu beschichten, um den abrasiven Verschleiß zu reduzieren. Dies bedarf eines aufwändigen und teuren Verfahrens. Zudem lassen sich die beschichteten Abschnitte nur unzureichend nachbearbeiten, also beispielsweise nachschleifen. Zur Vermeidung der Nachbearbeitung sind die Instrumente, insbesondere die Schneidabschnitte, oftmals als Einweg- oder "Semi-Reusable"-Instrumente ausgelegt, so dass auch hier hohe Kosten entstehen.

Näheres zum Stand der Technik enthält die US 2003/049185, die nach Ansicht des Europäischen Patentamtes ein elektrochirurgisches Instrument gemäß dem Oberbegriff des Anspruchs 1 offenbart.

Das US-Patent 6,267,761, die US-Patentanmeldungsschrift 2001/0037109 sowie das US-Patent 5,458,598 beschreiben ebenfalls elektrochirurgische Instrumente, die für die Beurteilung der Patentfähigkeit der hier beanspruchten Erfindungen nach Ansicht des Europäischen Patentamtes ebenfalls von Relevanz sind.

Vor diesem Hintergrund liegt der Erfindung die Aufgabe zugrunde, ein elektrochirurgisches Instrument zum Koagulieren der eingangs genannten Art dahin gehend weiterzubilden, dass eine für ein Schneiden von einem Gefäß oder einem Gewebe aufzubringende Kraft mittels eines Schneidinstruments und damit auch eine mechanische Belastung des Schneidinstruments, Insbesondere ein Verschleiß von Schneidabschnitten, reduziert wird.

Die Erfindung sieht ein elektrochirurgisches Instrument gemäß Anspruch 1 vor. Bevorzugte Ausführungsformen der Erfindung sind in den Unteransprüchen beschrieben.

Die beanspruchte Erfindung lässt sich durch die in dieser Schrift, d.h. in der vorliegenden Beschreibung sowie in den Zeichnungen, beschriebenen und dargestellten Ausführungsbeispiele besser verstehen. Im Allgemeinen spiegelt die vorliegende Offenbarung bevorzugte Ausführungsbeispiele der Erfindung. Dem aufmerksamen Leser wird jedoch auffallen, dass einige Aspekte der beschriebenen Ausführungsbeispiele über den Schutzumfang der Ansprüche hinausragen. Sofern die beschriebenen Ausführungsbeispiele tatsächlich über den Schutzumfang der Ansprüche hinausragen, sind die beschriebenen Ausführungsbeispiele als zusätzliches Hintergrundinformation zu betrachten und stellen keine Definition der Erfindung per se dar.

Insbesondere wird die Aufgabe durch ein elektrochirurgisches Instrument gelöst, das zwei gelenkig miteinander verbundene Branchen umfasst, die entsprechend einem Schneid - oder Klemmwerkzeug betätigbar sind. Ferner umfasst das Instrument einander gegenüberliegende Elektrodenteile mit Koagulationsflächen an distalen Enden der Branchen zum Fassen von einem Gefäß oder Gewebe und zum Durchleiten eines Koagulationsstromes durch das Gefäß oder Gewebe zu dessen Koagulation, sowie Stromzuführungseinrichtungen zum Zuführen des Koagulationsstromes zu den Elektrodenteilen von einem HF-Generator. Die Elektrodenteile weisen jeweils mindestens einen Spannbereich auf, derart, dass beim Einklemmen von dem Gefäß oder Gewebe dieses zwischen den Elektrodenteilen vorgespannt wird und an dem vorgespannten Gefäß oder Gewebe ein Schneidvorgang mittels eines Schneidinstruments durchführbar ist.

Ein wesentlicher Punkt der Erfindung liegt darin, dass das Gewebe durch die Spannbereiche des Koagulationsinstruments beidseitig in Richtung deren Endbereiche gezogen, d. h. gestreckt wird. Das unter Spannung stehende Gewebe ist dann mittels des mechanisch arbeitenden Schneidinstruments leichter zu schneiden, weil sich Fasern des Gewebes quer zu einer Schneidrichtung ausrichten und das Gewebe dabei dünner wird. Damit ist eine an dem vorgespannten Gewebe zu dessen vollständiger Durchtrennung aufzubringende Kraft erheblich reduziert und einer mechanischen Belastung des Schneidinstruments, insbesondere einem Verschleiß von Schneidabschnitten wird entgegengewirkt. Auch ist der Schneidvorgang durch den Operateur leichter zu bewerkstelligen und das Instrument einfacher zu handhaben.

In einer ersten bevorzugten Ausführungsform ist einer der Spannbereiche mindestens in einem ersten mittleren Abschnitt konvex, der ihm gegenüberliegende Spannbereich mindestens in einem zweiten mittleren Abschnitt konkav gekrümmt. Damit passen die Spannbereiche bei einem Zusammenführen der Branchen im Wesentlichen formschlüssig ineinander. Durch die gekrümmten Spannbereiche wird auf einfachste Art ein Spannen des Gewebes ermöglicht, weil dieses über den Spannbereichen gestreckt wird. Durch den Formschluss ist das Gewebe dann zwischen den Branchen in gespanntem Zustand sicher arretiert.

Die Begriffe "konvex" und "konkav" sind in diesem Zusammenhang nicht nur als im Bogen gerundet zu verstehen. Vielmehr ist mit den Termini jede Art von Erhabenheit bzw. Einbuchtung zu verstehen, also z. B. auch eine dachförmige Erhabenheit und dementsprechend eine V-förmige Einbuchtung.

In einer weiteren bevorzugten Ausführungsform ist einer der Spannbereiche mindestens in dem ersten mittleren Abschnitt konvex, der ihm gegenüberliegende Spannbereich mindestens in dem zweiten mittleren Abschnitt konkav gekrümmt. Dabei ist ein Krümmungsradius des konkav gekrümmten Spannbereichs mindestens im zweiten mittleren Abschnitt größer als ein Krümmungsradius des konvex gekrümmten Spannbereichs in dem ersten mittleren Abschnitt. Die Krümmungen verlaufen um Längsachsen der distalen Enden derart, dass das zwischen den distalen Enden gehaltene und senkrecht zu den Längsachsen verlaufende Gewebe mit zu dem ersten und zweiten mittleren Abschnitt hin steigender Pressung gehalten wird. Diese Ausführungsform hat den Vorteil, dass das Gewebe aufgrund der steigenden Pressung besonders sicher zwischen den Spannbereichen arretiert ist. Ein Abgleiten des einmal erfassten Gewebes wird somit ausgeschlossen. Zudem wird an den Bereichen hoher Pressung, also hohen Drucks, bedingt durch die hohe Klemmkraft ein sicheres Verschließen des Gewebes erreicht.

Eine erfindungsgemäße Lösung sieht vor, dass mindestens ein Spannbereich mindestens einen Durchlassbereich für das Schneidinstrument aufweist, so dass mindestens ein Abschnitt des Durchlassbereichs als Führungsspalt für das Schneidinstrument vorgesehen und an dem eingeklemmten Gewebe das Schneidinstrument zum Durchführen des Schneidvorgangs ansetzbar ist. Der Führungsspalt ermöglicht einen präzisen Schnitt an dem Gewebe, insbesondere bei mechanischen Schneidwerkzeugen.

Vorzugsweise teilt der Durchlassbereich die jeweiligen Elektrodenteile in mindestens zwei Bereiche, so dass die Elektrodenteile jeweils sich gegenüberliegende, parallel zueinander angeordnete Teilungsflächen aufweisen. Damit ist der Durchlassbereich über seinen gesamten Bereich als Führungsspalt nutzbar. Diese Art Führungsspalt ermöglicht einen äußerst präzisen Schnitt, weil das Schneidinstrument, insbesondere das mechanisch zu bedienende, besonders präzise führbar ist.

In einer weiteren bevorzugten Ausführungsform ist vorgesehen, dass der Durchlassbereich die jeweiligen Elektrodenteile in mindestens zwei Bereiche teilt, so dass die Elektrodenteile jeweils sich gegenüberliegende, sich in Richtung der Koagulationsflächen verjüngend zueinander angeordnete Teilungsflächen aufweisen. Da sich die Teilungsflächen der entsprechenden Elektrodenteile zu einem Schneidbereich an dem Gewebe hin annähern, ist dort weiterhin eine präzise Führung des Schneidinstruments gewährleistet. Der sich vom Schneidbereich weg gerichtete und aufgespreizte Teil des Durchlassbereichs eignet sich in besonderem Maße für eine Wiederaufbereitung, also Reinigung des Instruments nach erfolgtem Eingriff oder auch für das nachträgliche Aufbringen einer Beschichtung der Teilungsflächen mit einer beispielsweise verschleißresistenten Keramik, da durch die Ausgestaltung des Durchlassbereichs eine verbesserte Zugänglichkeit gewährleistet ist.

Vorzugsweise sind die Durchlassbereiche an den gegenüberliegenden Spannbereichen vorgesehen, wobei diese bei zusammengeführten Branchen im Wesentlichen fluchtend aneinander grenzen. Ist nur ein Durchlassbereich an einem Elektrodenteil ausgebildet, so eignet sich dieser insbesondere dafür, das Gewebe beispielsweise mittels eines chirurgischen Messers zu durchtrennen, wobei das Gewebe auf dem gegenüberliegenden Elektrodenteil vollständig, in gespanntem Zustand, aufliegt. Sind an beiden Elektrodenteilen Durchlassbereiche vorgesehen, so ist beispielsweise eine chirurgische Schere an dem koagulierten Gewebe ansetzbar und dieses auf einfache Weise zu durchtrennen. Zur Ausführung eines gut kalkulierbaren Schnittes sind die Durchlassbereiche vorzugsweise in den mittleren Abschnitten der Spannbereiche angeordnet.

In einer bevorzugten Ausführungsform ist das Schneidinstrument mit dem elektrochirurgischen Instrument verbunden ausgebildet. Beispielsweise befindet sich das Schneidinstrument innerhalb einer der Branchen und kann bei Bedarf in eine Schneidposition gebracht werden. Damit ist ein Instrumentenwechsel vermeidbar, so dass ein Operationsverlauf nicht unterbrochen werden muss.

Bei in das Koagulationsinstrument integriertem Schneidinstrument sind vorzugsweise beide Elektrodenteile mit dem Durchlassbereich ausgebildet, damit das Schneidinstrument das Gewebe ungehindert erreichen kann.

Ist das Schneidinstrument nicht integrativ mit dem elektrochirurgischen Instrument ausgebildet, so ist der Führungsspalt derart auszulegen, dass ein von außen kommendes Schneidinstrument bei hinreichend genauer Führung an dem vorgespannten Gewebe ansetzbar ist.

Eine vorteilhafte Ausführungsform sieht vor, dass das Schneidinstrument mechanisch und/oder elektrisch betätigbar ist. So kann beispielsweise eine an einem Schaft ausgebildete Klinge an dem elektrochirurgischen Instrument vorgesehen sein, die während der Koagulation in der Branche untergebracht ist und für den Schneidvorgang an das Gewebe herangeführt wird. Das Positionieren der Klinge oder eines sonstigen Schneidinstruments und auch ein Vorschub können hierbei selbsttätig erfolgen oder auch von dem Chirurgen mechanisch durchgeführt werden.

Eine erfindungsgemäße Lösung sieht vor, dass an dem einen Spannbereich und/oder an dem gegenüberliegenden Spannbereich ein einen Spanneffekt unterstützendes Oberflächenprofil ausgebildet ist. Das Profil ist vorzugsweise an Endbereichen des jeweiligen Spannbereichs ausgebildet und bewegt das Gewebe zusätzlich in einer durch die Spannbereiche definierte Zugrichtung bzw. verhindert ein Zurückweichen des Gewebes entgegen dieser Zugrichtung.

Vorzugsweise ist das den Spanneffekt unterstützende Oberflächenprofil als Sägezahnprofil ausgebildet. Zähne des Profils können beispielsweise so angeordnet sein, dass sie während des Zusammenführens der Branchen immer weiter in das Gewebe greifen und dieses in Zugrichtung mitnehmen. Damit wird die Spannung im Gewebe deutlich erhöht. Es ist allerdings darauf zu achten, dass durch das Profil eine Verletzung des Gewebes vermieden wird, so dass die Zähne vorzugsweise als abgerundete Noppen ausgebildet sind.

Vorzugsweise ist das Profil derart ausgestaltet, dass das Gewebe bei einem leichten Öffnen der Branchen durch das Profil in seiner gespannten Position gehalten wird. Das Profil fungiert demgemäß als eine Anordnung von Widerhaken.

In einer bevorzugten Ausführungsform ist das den Spanneffekt unterstützende Oberflächenprofil derart ausgebildet, dass zwischen den Elektrodenteilen mindestens eine Verengung vorgesehen ist. Dies ist insbesondere bei Elektrodenteilen mit gleichen Krümmungsradien zweckmäßig. Das heißt, die Koagulationsflächen der Elektrodenteile sind vorzugsweise an den beiden Endbereichen derart ausgestaltet, dass das Gewebe während des Zusammenführens der Branchen in Richtung der Endbereiche mitgenommen wird und bei zusammengeführten Branchen jeweils in einer Verengung gegenüber dem übrigen Bereich eingeklemmt ist. Die Verengung hat zusätzlich den Vorteil, dass die Koagulationsflächen im Wesentlichen glatt ausgebildet sein können und so leicht zu reinigen sind. Zudem wird die Verletzung des Gewebes aufgrund der glatten Oberfläche vermieden.

In einer vorteilhaften Ausführungsform ist an mindestens einer der Koagulationsflächen ein Isolationsabschnitt ausgebildet, so dass ein direkter elektrischer Kontakt zwischen den Koagulationsflächen vermeidbar ist. Aufgrund wärmeleitender Eigenschaften des Isolationsabschnittes wird auch an diesem eine Koagulation des Gewebes gewährleistet. Der Isolationsabschnitt ist je nach Ausgestaltung der Elektrodenteile an den Bereichen mindestens einer Koagulationsfläche vorzusehen, die der gegenüberliegenden Koagulationsfläche am nächsten sind. Dies ist insbesondere dann zu beachten, wenn die Spannbereiche und damit die Koagulationsflächen einen unterschiedlichen Krümmungsradius aufweisen. Vorzugsweise ist der Isolationsabschnitt dann an dem mittleren Bereich des Spannbereichs oder der Spannbereiche angeordnet und verhindert so einen Kurzschluss zwischen den Elektrodenteilen. Gleichzeitig wird der Spanneffekt durch den Isolationsabschnitt weiter begünstigt.

Ist der Isolationsabschnitt an den Bereichen mindestens einer Koagulationsfläche mit nächster Nähe zu der gegenüberliegenden Koagulationsfläche ausgebildet, kann er bündig mit der jeweiligen Koagulationsfläche abschließen. Der Flächenanteil der Koagulationsfläche, der den Bereich mit nächster Nähe zu der gegenüberliegenden Koagulationsfläche beschreibt, muss dann jedoch durchgängig aus isolierendem Material ausgebildet sein, so dass ein Kontakt zwischen den leitenden Bereichen der Koagulationsflächen vermieden wird. Bei den konvex bzw. konkav ausgebildeten, gegenüberliegenden Spannbereichen bzw. Koagulationsflächen mit unterschiedlichen Krümmungsradien, müsste der Isolationsabschnitt entlang einer Scheitellinie mindestens einer Koagulationsfläche angeordnet sein. Vorteilhafterweise ist der Isolationsabschnitt bei dieser Ausführungsform geschützt in dem jeweiligen Elektrodenteil und somit sicher vor Verschleiß untergebracht.

Alternativ ist es möglich, den Isolationsabschnitt derart auszubilden, dass dieser aus der jeweiligen Koagulationsfläche hervorsteht. In diesem Falle dient der Isolationsabschnitt nicht nur der Isolation, sondern auch dazu, das zu behandelnde Gewebe mehrfach zu knicken und so eine verbesserte Arretierung des Gewebes zwischen den distalen Enden des elektrochirurgischen Instruments zu erreichen.

In einer bevorzugten Ausführungsform ist der Isolationsabschnitt, d. h. der aus der jeweiligen Koagulationsfläche hervorstehende Isolationsabschnitt, aus mehreren Teilabschnitten ausgebildet. Dies ermöglicht eine besonders sichere Arretierung des Gewebes zwischen den Elektrodenteilen, weil das Gewebe mehrfach an Kanten des Isolationsabschnittes geknickt wird.

Eine erfindungsgemäße Lösung sieht vor, den Isolationsabschnitt selbst strukturiert auszubilden, um so eine optimale Arretierung des Gewebes zu erreichen.

Eine bevorzugte Ausführungsform sieht vor, den Isolationsabschnitt aus Keramik oder Diamant auszubilden. Vorteilhafterweise weisen Keramik und Diamant u. a. eine hohe Korrosionsbeständigkeit und eine hohe Verschleißfestigkeit gegenüber mechanischer Belastung auf.

In einer weiteren bevorzugten Ausführungsform ist der Isolationsabschnitt als das oder jedes, den oder jeden Spanneffekt unterstützendes Oberflächenprofil ausgebildet. Damit wird auf einfachste Weise sowohl der Kurzschluss zwischen den Elektrodenteilen vermieden, als auch das Spannen des Gewebes verstärkt.

Eine zwischen den Elektrodenteilen einen Kurzschluss verhindernde Einrichtung kann beispielsweise auch an den Branchen vorgesehen sein. Ist an diesen z. B. ein Abstandshalter angeordnet, können die Branchen nicht vollständig zusammengeführt werden, ein Abstand zwischen den Elektrodenteilen bleibt bestehen.

Elektrochirurgische Instrumente dieser Art können beispielsweise für den Einsatz am eröffneten Körper ausgebildet sein. Das Prinzip der mit einem Spannbereich ausgebildeten Elektrodenteile ist jedoch auch für in der Endoskopie eingesetzte Instrumente anwendbar. Die an den Branchen befestigten Elektrodenteile und ggf. das Schneidinstrument sind dann beispielsweise über einen an einem Schaft befestigten Handgriff zu betätigen oder aber es ist eine Steuerungseinheit vorgesehen, so dass eine Betätigung der Elektrodenteile und/oder des Schneidinstruments über diese gesteuert wird. So ist das elektrochirurgische Instrument vorzugsweise als laparoskopisches Instrument ausgebildet.

Weitere Ausführungsformen der Erfindung ergeben sich aus den Unteransprüchen.

Nachfolgend wird die Erfindung anhand von Ausführungsbeispielen beschrieben, die anhand der Abbildungen näher erläutert werden. Hierbei zeigen
- - Fig. 1: ein perspektivisch dargestelltes elektrochirurgisches Instrument mit einer erfindungsgemäßen Elektrodenanordnung in einer ersten bevorzugten Ausführungsform;
- - Fig. 2: eine schematisch im Schnitt dargestellte Elektrodenanordnung in Vorderansicht gemäß einer zweiten bevorzugten Ausführungsform;
- - Fig. 3: eine schematisch im Schnitt dargestellte Elektrodenanordnung in Vorderansicht gemäß einer dritten bevorzugten Ausführungsform;
- - Fig. 4: eine schematisch im Schnitt dargestellte Elektrodenanordnung in Vorderansicht gemäß einer vierten bevorzugten Ausführungsform;
- - Fig. 5: eine schematisch im Schnitt dargestellte Elektrodenanordnung in Vorderansicht gemäß einer fünften bevorzugten Ausführungsform.
- - Fig. 6: ein Spannbereich der Elektrodenanordnung gem. der ersten bevorzugten Ausführungsform aus Fig. 1.

In der nachfolgenden Beschreibung werden für gleiche und gleich wirkende Teile dieselben Bezugsziffern verwendet.

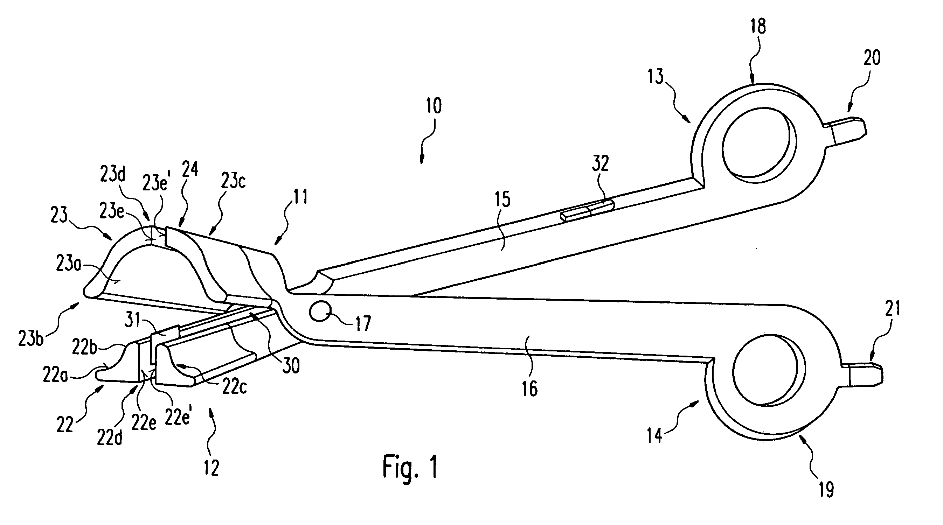

Fig. 1 zeigt ein perspektivisch dargestelltes elektrochirurgisches Instrument 10 mit einer erfindungsgemäßen Elektrodenanordnung in einer ersten bevorzugten Ausführungsform.

Das Instrument 10 ist für einen Eingriff am eröffneten Körper ausgebildet. In der Abbildung sind mit den Bezugsziffern 15 und 16 zwei Branchen des elektrochirurgischen Instruments 10 bezeichnet. Die beiden Branchen 15, 16 sind über eine Achse 17 miteinander verbunden und um diese verschwenkbar. Sie weisen mit Elektrodenteilen 22, 23 versehene distale Enden 11, 12 auf, wobei sich die Elektrodenteile 22, 23 einander gegenüberliegen. Mit Hilfe der Elektrodenteile 22, 23, die Koagulationsflächen 22a, 23a aufweisen, lässt sich beispielsweise ein Gefäß oder Gewebe fassen und durch Zuleitung von hochfrequentem Strom koagulieren. Ferner sind Griffteile 18, 19 vorgesehen, die an jeweilige proximale Enden 13, 14 der Branchen 15, 16 anschließen. Die proximalen Enden 13, 14 der Branchen 15, 16 enden jeweils in einem Stromanschlusselement bzw. einer Stromzuführungseinrichtung 20, 21 zum Anschließen des elektrochirurgischen Instruments 10 an einen (nicht dargestellten) HF-Generator, der eine HF-Spannung erzeugt, so dass der HF-Strom beispielsweise durch in dem Instrument 10 laufende elektrische Leitungen (nicht gezeigt) den Elektrodenteilen 22, 23 zugeführt werden kann.

Das elektrochirurgische Instrument 10 ist derart ausgebildet, dass sich ein Elektrodenteil 23 bei Zusammenführung der Branchen 15, 16 über das andere Elektrodenteil 22 stülpt, d. h., dieses abdeckt. Wie aus der Abbildung ersichtlich, sind die Elektrodenteile 22, 23 gekrümmt ausgebildet. Dabei weist ein Elektrodenteil 22 eine konvexe Krümmung 22b auf und das Elektrodenteil 23, das dem konvexen Elektrodenteil gegenüberliegt, weist eine konkave Krümmung 23b auf. Damit passen die Elektrodenteile 22, 23 bei zusammengeführten Branchen 15, 16 formschlüssig ineinander. Durch die gekrümmten Elektrodenteile 22, 23 wird das Gewebe in Richtung von Endbereichen der Elektrodenteile 22, 23 gezogen, d. h. gestreckt. Die Elektrodenteile 22, 23 bilden demgemäß Spannbereiche 22c, 23c aus. Damit lässt sich das Gewebe leichter schneiden, da sich Fasern des Gewebes quer zu einer Schneidrichtung ausrichten und das Gewebe dabei dünner wird. Durch den Formschluss ist das Gewebe dann zwischen den Branchen 15, 16 in gespanntem Zustand fixiert. In diesem Ausführungsbeispiel sind die Elektrodenteile 22, 23 im Wesentlichen vollständig als Spannbereiche 22c, 23c ausgebildet. Alternativ ist es möglich, dass nur Abschnitte der Elektrodenteile Spannbereiche ausbilden.

Die Spannbereiche 22c, 23c weisen Durchlassbereiche 22d, 23d auf, die einen Führungsspalt 24 für ein Schneidinstrument 30 ausbilden. An dem eingeklemmten Gewebe ist demgemäß das Schneidinstrument 30 zum Durchführen eines Schneidvorgangs ansetzbar.

Der Führungsspalt 24 ermöglicht zudem einen präzisen Schnitt an dem Gewebe, weil das Schneidinstrument 30 entlang dem Führungsspalt 24 führbar ist. Dies ist dann vorteilhaft, wenn das Schneidinstrument mechanisch bedient wird. Gleichzeitig verhindern die Spannbereiche 22c, 23c, dass das Gewebe in den Führungsspalt eindringt, da das Gewebe aufgrund der Spannung von und aus diesem weggezogen wird.

Da beide Spannbereiche 22c, 23c Durchlassbereiche 22d, 23d aufweisen, sind diese fluchtend aneinander angeordnet. Nur so lässt sich eine präzise Führung des Schneidinstruments 30 gewährleisten.

Wie in diesem Ausführungsbeispiel gezeigt, teilen die Durchlassbereiche 22d, 23d die jeweiligen Elektrodenteile 22, 23 in mindestens zwei Bereiche, so dass die Elektrodenteile 22, 23 jeweils sich gegenüberliegende, parallel zueinander angeordnete Teilungsflächen 22e, 22e' bzw. 23e, 23e' aufweisen. Damit sind die Durchlassbereiche 22d, 23d über ihre gesamte Länge als Führungsspalt 24 nutzbar. Diese Art Führungsspalt 24 ermöglicht einen äußerst präzisen Schnitt, weil das Schneidinstrument 30 besonders präzise führbar ist, insbesondere, wenn das Schneidinstrument mechanisch bedient wird.

Alternativ wäre es möglich, nur einen Durchlassbereich an einem Elektrodenteil auszubilden, so dass das Gewebe beispielsweise mittels eines chirurgischen Messers durchtrennbar ist. Dabei liegt das Gewebe auf dem gegenüberliegenden Elektrodenteil vollständig, in gespanntem Zustand, auf.

Das Schneidinstrument 30 weist eine Klinge 31 an einem Schaft auf und ist während einer Koagulationsphase innerhalb der Branche 15 untergebracht. Für den Schneidvorgang lässt sich das Schneidinstrument 30 an dem bereits koagulierten Gewebe positionieren und zum Durchtrennen des Gewebes mit einer definierten Vorschubgeschwindigkeit bewegen. Dies geschieht in diesem Ausführungsbeispiel beispielsweise durch eine (nicht gezeigte) das Schneidinstrument 30 ansteuernde Steuerungseinheit, die durch einen Fingerschalter 32 aktivierbar ist. Da das Schneidinstrument 30 in dem elektrochirurgischen Instrument 10 integriert ausgebildet ist, ist ein Instrumentenwechsel und damit eine Unterbrechung eines Operationsverlaufs vermeidbar.

Alternativ ist es möglich, das Schneidinstrument mechanisch durch den Anwender zu betätigen. Der Chirurg kann dann die Klinge 31 bei Bedarf durch die Branche 15 an und durch das Gewebe schieben.

Ist an dem elektrochirurgischen Instrument keine Vorrichtung zum Schneiden des Gewebes vorgesehen, so ist der Führungsspalt derart auszulegen, dass ein von außen kommendes Schneidinstrument, z. B. eine chirurgische Schere, bei hinreichend genauer Führung an dem vorgespannten Gewebe ansetzbar ist.

In der praktischen Anwendung ist an dem elektrochirurgischen Instrument 10 ein Abstandshalter (nicht gezeigt) oder dergleichen zwischen den Elektrodenteilen 22, 23 einen Abstand haltende Vorrichtung ausgebildet, damit ein unmittelbarer Kontakt der Koagulationsflächen 22a, 23a der Elektrodenteile 22, 23 und damit ein Kurzschluss vermeidbar ist. Der Abstandshalter kann beispielsweise an einer der Branchen 15, 16 ausgebildet sein.

Alternativ ist es möglich, den Abstandshalter als Isolationsabschnitt an den Elektrodenteilen vorzusehen. Aufgrund wärmeleitender Eigenschaften des Isolationsabschnittes wird auch an diesem eine Koagulation gewährleistet.

Das in Fig. 1 gezeigte elektrochirurgische Instrument 10 ist, wie bereits oben erwähnt, für den Einsatz am eröffneten Körper ausgebildet. Das Prinzip der mit den Spannbereichen ausgebildeten Elektrodenteilen ist ebenso für die Endoskopie anzuwenden. Die an den Branchen befestigten Elektrodenteile und ggf. das Schneidinstrument sind dann beispielsweise über einen an einem Schaft befestigten Handgriff zu betätigen oder aber es ist eine Steuerungseinheit vorgesehen, so dass eine Betätigung der Elektrodenteile und/oder des Schneidinstruments über diese gesteuert wird.

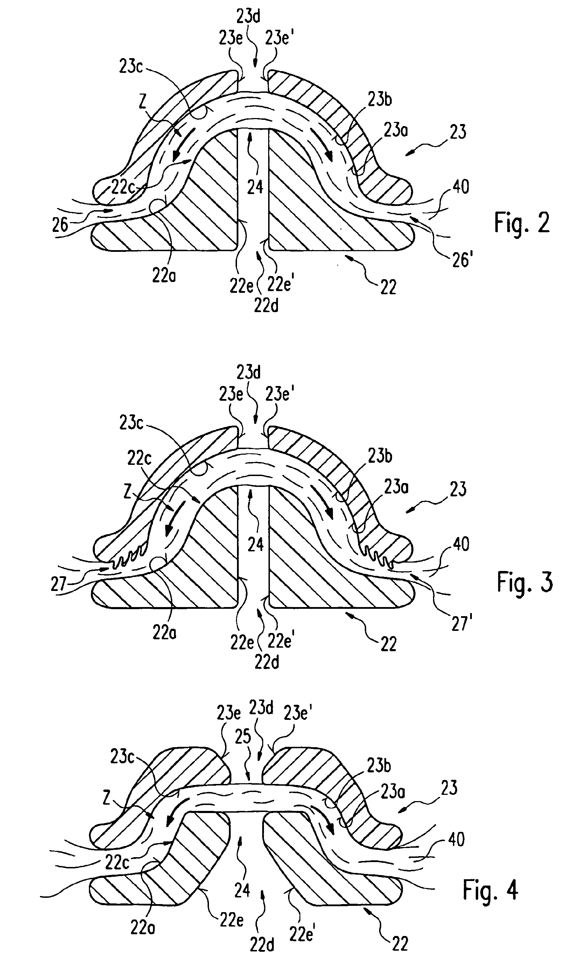

Fig. 2 und 3 zeigen jeweils eine stark vergrößerte Vorderansicht einer Elektrodenanordnung im Schnitt in einer zweiten bzw. in einer dritten Ausführungsform. Die Elektrodenteile 22, 23 entsprechen im Wesentlichen der Ausgestaltung der in Fig. 1 gezeigten. Auch weisen diese als Führungsspalt 24 für ein Schneidinstrument dienende Durchlassbereiche 22d, 23d wie in Fig. 1 beschrieben auf. An mindestens einem Spannbereiche ist ein einen Spanneffekt von eingeklemmtem Gewebe 40 unterstützendes Oberflächenprofil ausgebildet. Das Profil ist vorzugsweise an beiden Endbereichen des jeweiligen Spannbereichs bzw. der jeweiligen Spannbereiche 22c, 23c ausgebildet und bewegt das Gewebe 40 zusätzlich in einer durch die Spannbereiche 22c, 23c definierte Zugrichtung Z bzw. verhindert ein Zurückweichen des Gewebes 40 entgegen dieser Zugrichtung Z.

Wie in Fig. 2 gezeigt, sind Koagulationsflächen 22a, 23a der Elektrodenteile 22, 23 an deren beiden Endbereichen derart ausgestaltet, dass das Gewebe 40 während eines Zusammenführens von Branchen 15, 16 in Richtung der Endbereiche mitgenommen wird und bei zusammengeführten Branchen 15, 16 jeweils in einer Verengung 26, 26' gegenüber dem übrigen Bereich eingeklemmt ist. Die Verengung 26, 26' hat zusätzlich den Vorteil, dass die Koagulationsflächen 22a, 23a im Wesentlichen glatt ausgebildet sein können und so leicht zu reinigen sind. Zudem wird eine Verletzung des Gewebes aufgrund der glatten Oberflächen vermieden.

In Fig. 3 weist das mit einer konkaven Krümmung 23b ausgebildete Elektrodenteil 23 an Endbereichen ein sägezahnartiges Profil 27, 27' auf. Die Zähne, können beispielsweise so angeordnet sein, dass sie während einem Zusammenführen der Branchen immer weiter in das Gewebe 40 greifen und dieses in Zugrichtung Z mitnehmen. Damit wird die Spannung im Gewebe 40 deutlich erhöht. Es ist allerdings darauf zu achten, dass durch das Profil 27, 27' eine Verletzung des Gewebes 40 vermieden wird, so dass die Zähne vorzugsweise als Noppen ausgebildet sind.

Vorzugsweise sind die Noppen derart angeordnet, dass das Gewebe 40 bei einem leichten Öffnen der Branchen durch das Profil 27, 27' in seiner gespannten Position gehalten wird. Das Profil 27, 27' fungiert demgemäß als eine Anordnung von Widerhaken.

Fig. 4 zeigt eine vergrößerte Vorderansicht einer Elektrodenanordnung im Schnitt in einer vierten Ausführungsform. Dabei teilen Durchlassbereiche 22d, 23d die jeweiligen Elektrodenteile 22, 23 in mindestens zwei Bereiche derart, dass die Elektrodenteile 22, 23 jeweils sich gegenüberliegende, sich in Richtung zu Koagulationsflächen 22a, 23a hin verjüngend zueinander angeordnete Teilungsflächen 22e, 22e' bzw. 23e, 23e' aufweisen. Da sich die Teilungsflächen 22e, 22e', 23e, 23e' der entsprechenden Elektrodenteile 22, 23 zu einem Schneidbereich 25 hin annähern, ist dort weiterhin eine präzise Führung des Schneidinstruments 30 gewährleistet. Der sich vom Schneidbereich 25 weg gerichtete Teil der Durchlassbereiche 22d, 23d eignet sich in besonderem Maße für eine Wiederaufbereitung, also Reinigung des Instruments 10 nach erfolgtem Eingriff oder auch für das nachträgliche Aufbringen einer Beschichtung der Teilungsflächen 22e, 22e', 23e, 23e' mit einer beispielsweise verschleißresistenten Keramikschicht, da durch die Ausgestaltung der Durchlassbereiche 22d, 23d eine verbesserte Zugänglichkeit gewährleistet ist.

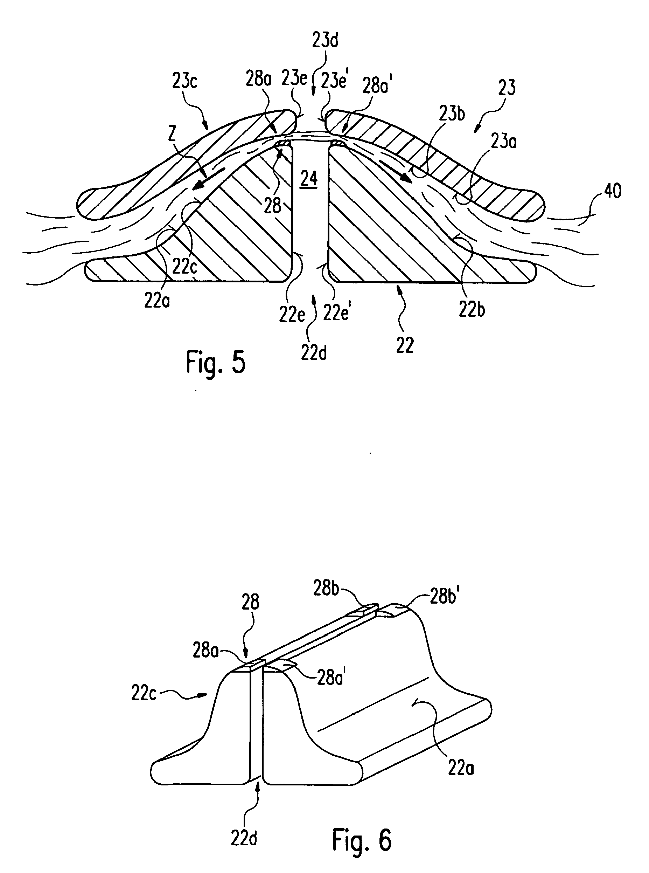

Fig. 5 zeigt eine vergrößerte Vorderansicht einer Elektrodenanordnung im Schnitt in einer fünften Ausführungsform. Diese Ausführungsform entspricht im Wesentlichen der in den Fig. 1 und 2 gezeigten. Der Unterschied besteht darin, dass Spannbereiche 22c, 23c hier mit unterschiedlichen Krümmungsradien ausgebildet sind; der Krümmungsradius des konkav ausgebildeten Spannbereichs 23c ist größer als der Krümmungsradius des konvex ausgebildeten. Damit sind auch die jeweiligen Koagulationsflächen 22a, 23a entsprechend gekrümmt. Die Krümmungen 22b, 23b verlaufen um Längsachsen der distalen Enden derart, dass ein zwischen den distalen Enden 11, 12 gehaltenes und senkrecht zu den Längsachsen verlaufendes Gewebe 40 mit zu mittleren Abschnitten der Spannbereiche 22c, 23c hin steigender Pressung gehalten wird. Vorteilhafterweise wird hier ein einmal gespanntes Gewebe 40 aufgrund der steigenden Pressung besonders sicher zwischen den Spannbereichen 22c, 23c arretiert. Ein Abgleiten des einmal erfassten Gewebes 40 aus den Elektrodenteilen 22, 23 ist somit ausgeschlossen. Zudem wird an den Bereichen hoher Pressung, also hohen Drucks, bedingt durch die hohe Klemmkraft, ein sicheres Verschließen des Gewebes 40 erreicht.

Unmittelbar benachbart einem Durchlassbereich 22d ist ein aus zwei Teilabschnitten 28a, 28a' ausgebildeter, hervorstehender Isolationsabschnitt 28 an dem durch den Durchlassbereich 22d in zwei Bereiche aufgeteilten, konvex ausgebildeten Spannbereich 22c vorgesehen. Vorzugsweise verlaufen die Teilabschnitte 28a, 28a' des Isolationsabschnitts 28 parallel einer Scheitellinie des Spannbereichs 22c über das Elektrodenteil 22. Damit wird ein Kurzschluss zwischen den Elektrodenteilen 22, 23 bei einem Zusammenführen derselben verhindert. Die Teilabschnitte 28a, 28a' des Isolationsabschnittes 28 unterstützen einerseits die Spannwirkung des Spannbereichs 22 und ermöglichen andererseits eine Knickung des eingespannten Gewebes 40. Damit ist dessen zuverlässige Arretierung zwischen den Elektrodenteilen 22, 23 gewährleistet.

Grundsätzlich ist der Isolationsabschnitt je nach Ausgestaltung der Elektrodenteile an Bereichen mindestens eines Spannbereichs bzw. einer Koagulationsfläche vorzusehen, die der gegenüberliegenden Koagulationsfläche am nächsten sind. Dies ist insbesondere dann zu beachten, wenn die Spannbereiche und damit die Koagulationsflächen einen unterschiedlichen Krümmungsradius aufweisen. Vorzugsweise ist der Isolationsabschnitt dann an einem mittleren Bereich des Spannbereichs angeordnet und verhindert so einen Kurzschluss zwischen den Elektrodenteilen.

Ist der Isolationsabschnitt an den Bereichen mindestens einer Koagulationsfläche mit nächster Nähe zu der gegenüberliegenden Koagulationsfläche ausgebildet, kann er bündig mit der jeweiligen Koagulationsfläche abschließen. Dazu ist es jedoch erforderlich, dass ein Flächenanteil der Koagulationsfläche, der den Bereich mit nächster Nähe zu der gegenüberliegenden Koagulationsfläche beschreibt, vollständig aus isolierendem Material ausgebildet ist. Nur so lässt sich ein Kontakt zwischen den leitenden Bereichen der Koagulationsflächen vermeiden. Bei den konvex bzw. konkav ausgebildeten Spannbereichen mit unterschiedlichen Krümmungsradien müsste der Isolationsabschnitt entlang einer Scheitellinie mindestens eines Spannbereichs, zwischen den Koagulationsflächen angeordnet sein. In dieser Ausführungsform ist der Isolationsabschnitt geschützt in dem jeweiligen Elektrodenteil untergebracht, so dass einem Verschleiß des Isolationsabschnitts entgegengewirkt wird.

Fig. 6 zeigt eine perspektivische Ansicht eines konvex ausgebildeten Spannbereichs 22c gem. Fig. 1. Dabei ist ein Isolationsabschnitt 28 vorgesehen, der aus vier Teilabschnitten 28a, 28a', 28b, 28b' besteht, wobei jeweils zwei Teilabschnitte an den jeweiligen Bereichen des Spannbereichs 22c bzw. des Elektrodenteils 22 ausgebildet sind. Mittels mehrerer Teilabschnitte 28a, 28a', 28b, 28b' lässt sich das zu behandelnde Gewebe zuverlässig arretieren, weil es über Kanten der Teilabschnitte 28a, 28a', 28b, 28b' des Isolationsabschnittes 28 knickbar ist. Zudem wird die Koagulationsfläche 22a an nur wenigen Bereichen durch den Isolationsabschnitt 28 verdeckt.

Alternativ ist es möglich, den Isolationsabschnitt strukturiert auszubilden, um damit die Arretierung des Gewebes weiter zu verbessern.

Vorzugweise ist der Isolationsabschnitt aus Keramik oder Diamant ausgebildet, da beide Materialien u. a. eine hohe Korrosionsbeständigkeit und eine hohe Verschleißfestigkeit gegenüber mechanischer Belastung aufweisen.

Vorteilhafterweise kann der Isolationsabschnitt als ein einen Spanneffekt der Spannbereiche unterstützendes Oberflächenprofil ausgebildet sein. Damit wird auf einfachste Weise sowohl die Vermeidung des Kurzschlusses zwischen den Elektrodenteilen vermieden, als auch das Spannen des Gewebes verstärkt.

An dieser Stelle sei darauf hingewiesen, dass alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

### Bezugszeichenliste

- 10: Elektrochirurgisches Instrument
- 11: Distales Ende
- 12: Distales Ende
- 13: Proximales Ende
- 14: Proximales Ende
- 15: Branche
- 16: Branche
- 17: Achse
- 18: Griffteil
- 19: Griffteil
- 20: Stromanschlusselement, Stromzuführungseinrichtung
- 21: Stromanschlusselement, Stromzuführungseinrichtung
- 22: Elektrodenteil
- 22a: Koagulationsfläche
- 22b: Konvexe Krümmung
- 22c: Spannbereich
- 22d: Durchlassbereich
- 22e, 22e': Teilungsfläche
- 23: Elektrodenteil
- 23a: Koagulationsfläche
- 23b: Konkave Krümmung
- 23c: Spannbereich
- 23d: Durchlassbereich
- 23e, 23e': Teilungsfläche
- 24: Führungsspalt
- 25: Schneidbereich
- 26, 26': Verengung
- 27, 27': Profil
- 28: Isolationsabschnitt
- 28a, 28a': Teilabschnitt des Isolationsabschnitts
- 28b, 28b': Teilabschnitt des Isolationsabschnitts
- 30: Schneidinstrument
- 31: Klinge
- 32: Fingerschalter
- 40: Gewebe, Gefäß
- Z: Zugrichtung

## Patentansprüche

1. Elektrochirurgisches Instrument mit
- zwei gelenkig miteinander verbundenen Branchen (15, 16), die entsprechend einem Schneid- oder Klemmwerkzeug betätigbar sind,
- einander gegenüberliegenden Elektrodenteilen (22, 23) mit Koagulationsflächen (22a, 23a) an distalen Enden (11, 12) der Branchen (15, 16) zum Fassen von einem Gefäß oder Gewebe (40) und zum Durchleiten eines Koagulationsstromes durch das Gefäß oder Gewebe (40) zu dessen Koagulation,
- Stromzuführungseinrichtungen (20, 21) zum Zuführen des Koagulationsstromes zu den Elektrodenteilen (22, 23) von einem HF-Generator, und
- einem Schneidinstrument (30), wobei
die Elektrodenteile (22, 23) jeweils mindestens einen Spannbereich (22c, 23c) aufweisen, derart, dass beim Einklemmen von dem Gefäß oder Gewebe (40) dieses zwischen den Elektrodenteilen (22, 23) vorgespannt wird und an dem vorgespannten Gefäß oder Gewebe (40) ein Schneidvorgang mittels des Schneidinstruments (30) durchführbar ist,
**dadurch gekennzeichnet, dass**
eine der Branchen (15) zur Unterbringung des Schneidinstruments (30) an einer Position innerhalb der Branche (15) konfiguriert ist, und
das Schneidinstrument (30) zwischen der Position innerhalb der Branche (15) und Positionen bewegbar ist, bei denen das Schneidinstrument (30) das vorgespannte Gewebe bzw. Gefäß durchtrennt.

2. Elektrochirurgisches Instrument nach Anspruch 1
**dadurch gekennzeichnet, dass**
einer der Spannbereiche (22c) mindestens in einem ersten mittleren Abschnitt konvex, der ihm gegenüberliegende Spannbereich (23c) mindesten in einem zweiten mittleren Abschnitt konkav gekrümmt ist, so dass bei einem Zusammenführen der Branchen (15, 16) die Spannbereiche (22c, 23c) im Wesentlichen formschlüssig ineinander passen.

3. Elektrochirurgisches Instrument nach Anspruch 1 oder 2, insbesondere nach Anspruch 1,
**dadurch gekennzeichnet, dass**
einer der Spannbereiche (22c) mindestens in dem ersten mittleren Abschnitt konvex, der ihm gegenüberliegende Spannbereich (23c) mindestens in dem zweiten mittleren Abschnitt konkav gekrümmt ist, wobei ein Krümmungsradius des konkav gekrümmten Spannbereichs (23c) mindestens in dem zweiten mittleren Abschnitt größer ist als ein Krümmungsradius des konvex gekrümmten Spannbereichs (22c) in dem ersten mittleren Abschnitt und wobei die Krümmungen (22b, 23b) um Längsachsen der distalen Enden (11, 12) derart verlaufen, dass das zwischen den distalen Enden (11, 12) gehaltene und senkrecht zu den Längsachsen verlaufende Gewebe (40) mit zu dem ersten und zweiten mittleren Abschnitt hin steigender Pressung gehalten wird.

4. Elektrochirurgisches Instrument nach einem der vorhergehenden Ansprüche,
**dadurch gekennzeichnet, dass**
mindestens ein Spannbereich (22c, 23c) mindestens einen Durchlassbereich (22d, 23d) für das Schneidinstrument (30) aufweist, so dass mindestens ein Abschnitt des Durchlassbereichs (22d, 23d) als Führungsspalt (24) für das Schneidinstrument (30) vorgesehen und an dem eingeklemmten Gewebe (40) das Schneidinstrument (30) zum Durchführen des Schneidvorgangs ansetzbar ist.

5. Elektrochirurgisches Instrument nach einem der vorhergehenden Ansprüche, insbesondere nach Anspruch 4,
**dadurch gekennzeichnet, dass**
der Durchlassbereich (22d, 23d) die jeweiligen Elektrodenteile (22, 23) in mindestens zwei Bereiche teilt, so dass die Elektrodenteile (22, 23) jeweils sich gegenüberliegende, parallel zueinander angeordnete Teilungsflächen (22e, 22e', 23e, 23e') aufweisen.

6. Elektrochirurgisches Instrument nach einem der vorhergehenden Ansprüche, insbesondere nach Anspruch 4,
**dadurch gekennzeichnet, dass**
der Durchlassbereich (22d, 23d) die jeweiligen Elektrodenteile (22, 23) in mindestens zwei Bereiche teilt, so dass die Elektrodenteile (22, 23) jeweils sich gegenüberliegende, sich in Richtung der Koagulationsflächen (22a, 23a) verjüngend zueinander angeordnete Teilungsflächen (22e, 22e', 23e, 23e') aufweisen.

7. Elektrochirurgisches Instrument nach einem der vorhergehenden Ansprüche, insbesondere nach einem der Ansprüche 4 bis 6,
**dadurch gekennzeichnet, dass**
Durchlassbereiche (22d, 23d) an den gegenüberliegenden Spannbereichen (22c, 23c) vorgesehen sind, die bei zusammengeführten Branchen (15, 16) im Wesentlichen fluchtend aneinander grenzen.

8. Elektrochirurgisches Instrument nach einem der vorhergehenden Ansprüche,
**dadurch gekennzeichnet, dass**
das Schneidinstrument (30) mit dem elektrochirurgischen Instrument (10) verbunden ausgebildet ist.

9. Elektrochirurgisches Instrument nach einem der vorhergehenden Ansprüche,
**dadurch gekennzeichnet, dass**
das Schneidinstrument (30) mechanisch und/oder elektrisch betätigbar ist.

10. Elektrochirurgisches Instrument nach einem der vorhergehenden Ansprüche,
**dadurch gekennzeichnet, dass**
an dem einen Spannbereich und/oder an dem gegenüberliegenden Spannbereich ein einen Spanneffekt unterstützendes Oberflächenprofil (27, 27') ausgebildet ist.

11. Elektrochirurgisches Instrument nach einem der vorhergehenden Ansprüche, insbesondere nach Anspruch 10,
**dadurch gekennzeichnet, dass**
das den Spanneffekt unterstützende Oberflächenprofil (27, 27') als Sägezahnprofil ausgebildet ist.

12. Elektrochirurgisches Instrument nach einem der vorhergehenden Ansprüche, insbesondere nach einem der Ansprüche 10 oder 11,
**dadurch gekennzeichnet, dass**
das den Spanneffekt unterstützende Oberflächenprofil (27, 27') derart ausgebildet ist, dass zwischen den Elektrodenteilen (22, 23) mindestens eine Verengung (26, 26') vorgesehen ist.

13. Elektrochirurgisches Instrument nach einem der vorhergehenden Ansprüche,
**dadurch gekennzeichnet, dass**
an mindestens einer der Koagulationsflächen ein Isolationsabschnitt (28) ausgebildet ist, so dass ein direkter elektrischer Kontakt zwischen den Koagulationsflächen (22a, 23a) vermeidbar ist.

14. Elektrochirurgisches Instrument nach einem der vorhergehenden Ansprüche, insbesondere nach Anspruch 13,
**dadurch gekennzeichnet, dass**
der Isolationsabschnitt (28) aus mehreren Teilabschnitten (28a, 28a', 28b, 28b') ausgebildet ist.

15. Elektrochirurgisches Instrument nach einem der vorhergehenden Ansprüche, insbesondere nach Anspruch 13 oder 14,
**dadurch gekennzeichnet, dass**
der Isolationsabschnitt (28) strukturiert ausgebildet ist.

16. Elektrochirurgisches Instrument nach einem der vorhergehenden Ansprüche, insbesondere nach einem der Ansprüche 13 bis 15,
**dadurch gekennzeichnet, dass**
der Isolationsabschnitt (28) aus Keramik oder Diamant ausgebildet ist.

17. Elektrochirurgisches Instrument nach einem der vorhergehenden Ansprüche, insbesondere nach einem der Ansprüche 13 bis 16,
**dadurch gekennzeichnet, dass**
der Isolationsabschnitt (28) als das oder jedes, den oder jeden Spanneffekt unterstützende Oberflächenprofil (27, 27') ausgebildet ist.

18. Elektrochirurgisches Instrument nach einem der vorhergehenden Ansprüche,
**dadurch gekennzeichnet, dass**
das Instrument als laparoskopisches Instrument ausgebildet ist.

## Claims

1. Electrosurgical instrument comprising
- two branches (15, 16) which are interconnected in a hinged manner and can be actuated in a corresponding manner to a cutting or clamping tool,
- electrode parts (22, 23) with coagulation areas (22a, 23a), situated opposite to one another at distal ends (11, 12) of the branches (15, 16), for gripping a vessel or tissue (40) and for passing coagulation current through the vessel or tissue (40) in order to coagulate the latter,
- current supply apparatuses (20, 21) for supplying the coagulation current to the electrode parts (22, 23) from an RF generator, and
- a cutting instrument (30), wherein
the electrode parts (22, 23) respectively have at least one tensioning region (22c, 23c) such that, when the vessel or tissue (40) is clamped, the latter is pretensioned between the electrode parts (22, 23) and a cutting process can be carried out on the pretensioned vessel or tissue (40) by means of the cutting instrument (30),
**characterized in that**
one of the branches (15) is configured to house the cutting instrument (30) at a position within the branch (15) and
the cutting instrument (30) can be moved between the position within the branch (15) and positions in which the cutting instrument (30) severs the pretensioned tissue or vessel.

2. Electrosurgical instrument according to Claim 1,
**characterized in that**
one of the tensioning regions (22c) has a convex curvature at least in a first central section, the tensioning region (23c) situated opposite thereto has a concave curvature at least in a second central section such that when the branches (15, 16) are brought together, the tensioning regions (22c, 23c) substantially have an interlocking fit into one another.

3. Electrosurgical instrument according to Claim 1 or 2, more particularly according to Claim 1,
**characterized in that**
one of the tensioning regions (22c) has a convex curvature at least in the first central section, the tensioning region (23c) situated opposite thereto has a concave curvature at least in the second central section, wherein a radius of curvature of the concavely curved tensioning region (23c) at least in the second central section is greater than a radius of curvature of the convexly curved tensioning region (22c) in the first central section and wherein the curves (22b, 23b) run about the longitudinal axes of the distal ends (11, 12) such that the tissue (40), which is held between the distal ends (11, 12) and runs perpendicularly to the longitudinal axes, is held with pressing that increases to the first and second central section.

4. Electrosurgical instrument according to one of the preceding claims,
**characterized in that**
at least one tensioning region (22c, 23c) has at least one passage region (22d, 23d) for the cutting instrument (30) such that at least one section of the passage region (22d, 23d) is provided as guide gap (24) for the cutting instrument (30) and the cutting instrument (30) can be placed onto the clamped tissue (40) for carrying out the cutting process.

5. Electrosurgical instrument according to one of the preceding claims, more particularly according to Claim 4,
**characterized in that**
the passage region (22d, 23d) subdivides the respective electrode parts (22, 23) into at least two regions such that the electrode parts (22, 23) respectively have opposing dividing areas (22e, 22e', 23e, 23e') which are arranged parallel to one another.

6. Electrosurgical instrument according to one of the preceding claims, more particularly according to Claim 4,
**characterized in that**
the passage region (22d, 23d) subdivides the respective electrode parts (22, 23) into at least two regions such that the electrode parts (22, 23) respectively have opposing dividing areas (22e, 22e', 23e, 23e') which are arranged tapering with respect to one another in the direction of the coagulation areas (22a, 23a).

7. Electrosurgical instrument according to one of the preceding claims, more particularly according to one of Claims 4 to 6,
**characterized in that**
passage regions (22d, 23d) are provided on the opposing tensioning regions (22c, 23c), which adjoin one another in a substantially flush fashion when the branches (15, 16) are brought together.

8. Electrosurgical instrument according to one of the preceding claims,
**characterized in that**
the cutting instrument (30) is formed connected to the electrosurgical instrument (10).

9. Electrosurgical instrument according to one of the preceding claims,
**characterized in that**
the cutting instrument (30) can be actuated mechanically and/or electrically.

10. Electrosurgical instrument according to one of the preceding claims,
**characterized in that**
a surface profile (27, 27') which supports a tensioning effect is formed on the one tensioning region and/or on the opposing tensioning region.

11. Electrosurgical instrument according to one of the preceding claims, more particularly according to Claim 10,
**characterized in that**
the surface profile (27, 27') supporting the tensioning effect is formed as saw-tooth profile.

12. Electrosurgical instrument according to one of the preceding claims, more particularly according to one of Claims 10 or 11,
**characterized in that**
the surface profile (27, 27') supporting .the tensioning effect is formed such that at least one narrowing (26, 26') is provided between the electrode parts (22, 23).

13. Electrosurgical instrument according to one of the preceding claims,
**characterized in that**
an insulation section (28) is formed on at least one of the coagulation areas such that direct electrical contact can be avoided between the coagulation areas (22a, 23a).

14. Electrosurgical instrument according to one of the preceding claims, more particularly according to Claim 13,
**characterized in that**
the insulation section (28) is formed from a plurality of partial sections (28a, 28a', 28b, 28b').

15. Electrosurgical instrument according to one of the preceding claims, more particularly according to Claim 13 or 14,
**characterized in that**
the insulation section (28) has a structured form.

16. Electrosurgical instrument according to one of the preceding claims, more particularly according to one of Claims 13 to 15,
**characterized in that**
the insulation section (28) is formed from ceramic or diamond.

17. Electrosurgical instrument according to one of the preceding claims, more particularly according to one of Claims 13 to 16,
**characterized in that**
the insulation section (28) is formed as the or every surface profile (27, 27') which supports the or every tensioning effect.

18. Electrosurgical instrument according to one of the preceding claims,
**characterized in that**
the instrument is embodied as laparoscopic instrument.

## Revendications

1. Instrument électrochirurgical doté :
de deux branches (15, 16) reliées entre elles de façon articulée et pouvant être actionnées conformément à un outil de coupe ou de serrage ;
de parties d'électrode (22, 23) opposées dotées de surfaces de coagulation (22a, 23a) au niveau des extrémités distales (11, 12) des branches (15, 16) pour saisir un vaisseau ou un tissu (40) et pour guider un courant de coagulation à travers le vaisseau ou le tissu (40) en vue de permettre sa coagulation ;
de dispositifs de conduction de courant (20, 21) servant à conduire le courant de coagulation jusqu'aux parties d'électrode (22, 23) en partant d'un générateur HF ; et
d'un instrument de coupe (30) ;
les parties d'électrode (22, 23) comportant respectivement au moins une région de serrage (22c, 23c) telle que lors du pincement du vaisseau ou du tissu (40), celui-ci est précontraint entre les parties d'électrode (22, 23) et qu'un processus de coupe peut être réalisé au niveau du vaisseau ou du tissu (40) précontraint à l'aide de l'instrument de coupe (30) ;
**caractérisé en ce que** :
une des branches (15) est configurée pour recevoir l'instrument de coupe (30) dans une certaine position à l'intérieur de la branche (15) ; et
l'instrument de coupe (30) peut être déplacé entre la position prévue à l'intérieur de la branche (15) et des positions dans lesquelles l'instrument de coupe (30) coupe en deux le tissu ou le vaisseau précontraint.

2. Instrument électrochirurgical selon la revendication 1, **caractérisé en ce qu'**une des régions de serrage (22c) est coudée de façon convexe au moins dans une première section centrale située en vis-à-vis de la région de serrage (23c) coudée de façon concave au moins dans une deuxième section centrale, de sorte qu'en cas de guidage conjoint des branches (15, 16), les régions de serrage (22c, 23c) s'ajustent pour l'essentiel l'une dans l'autre par complémentarité de formes.

3. Instrument électrochirurgical selon la revendication 1 ou 2, notamment selon la revendication 1, **caractérisé en ce qu'**une des régions de serrage (22c) est coudée de façon convexe au moins dans une première section centrale située en vis-à-vis de la région de serrage (23c) coudée de façon concave au moins dans une deuxième section centrale, un rayon de courbure de la région de serrage (23c) coudée de façon concave étant plus grand au moins dans la deuxième section centrale qu'un rayon de courbure de la région de serrage (22c) coudée de façon convexe dans la première section centrale et les courbures (22b, 23b) s'étendant autour des axes longitudinaux des extrémités distales (11, 12) de telle sorte que le tissu (40) maintenu entre les extrémités distales (11, 12) et s'étendant perpendiculairement aux axes longitudinaux est maintenu avec une pression croissante en direction de la première et de la deuxième section centrale.

4. Instrument électrochirurgical selon l'une quelconque des revendications précédentes, **caractérisé en ce qu'**au moins une région de serrage (22c, 23c) comporte au moins une région de passage (22d, 23d) pour l'instrument de coupe (30), de sorte qu'au moins une section de la région de passage (22d, 23d) prend la forme d'une fente de guidage (24) pour l'instrument de coupe (30) et que l'instrument de coupe (30) peut être placé contre le tissu (40) coincé pour réaliser le processus de coupe.

5. Instrument électrochirurgical selon l'une quelconque des revendications précédentes, notamment selon la revendication 4, **caractérisé en ce que** la région de passage (22d, 23d) divise les parties d'électrode (22, 23) respectives en au moins deux régions, de sorte que les parties d'électrode (22, 23) présentent respectivement des surfaces de séparation (22e, 22e', 23e, 23e') opposées, disposées parallèlement les unes par rapport aux autres.

6. Instrument électrochirurgical selon l'une quelconque des revendications précédentes, notamment selon la revendication 4, **caractérisé en ce que** la région de passage (22d, 23d) divise les parties d'électrode (22, 23) respectives en au moins deux régions, de sorte que les parties d'électrode (22, 23) comportent respectivement des surfaces de séparation (22e, 22e', 23e, 23e') opposées, disposées les unes par rapport aux autres de façon à se rétrécir en direction des surfaces de coagulation (22a, 23a).

7. Instrument électrochirurgical selon l'une quelconque des revendications précédentes, notamment selon l'une quelconque des revendications 4 à 6, **caractérisé en ce que** des régions de passage (22d, 23d) sont prévues au niveau des régions de serrage (22c, 23c) opposées, lesdites régions se touchant les unes les autres pour l'essentiel de façon alignée lorsque les branches (15, 16) sont guidées conjointement.

8. Instrument électrochirurgical selon l'une quelconque des revendications précédentes, **caractérisé en ce que** l'instrument de coupe (30) est réalisé de façon à être relié à l'instrument électrochirurgical (10).

9. Instrument électrochirurgical selon l'une quelconque des revendications précédentes, **caractérisé en ce que** l'instrument de coupe (30) peut être actionné de façon mécanique et/ou électrique.

10. Instrument électrochirurgical selon l'une quelconque des revendications précédentes, **caractérisé en ce qu'**un profil de surface (27, 27') contribuant à l'effet de serrage est réalisé au niveau d'une région de serrage et/ou de la région de serrage opposée.

11. Instrument électrochirurgical selon l'une quelconque des revendications précédentes, notamment selon la revendication 10, **caractérisé en ce que** le profil de surface (27, 27') contribuant à l'effet de serrage prend la forme d'un profil en dents de scie.

12. Instrument électrochirurgical selon l'une quelconque des revendications précédentes, notamment selon l'une quelconque des revendications 10 ou 11, **caractérisé en ce que** le profil de surface (27, 27') contribuant à l'effet de serrage est réalisé de telle sorte qu'au moins un rétrécissement (26, 26') est prévu entre les parties d'électrode (22, 23).

13. Instrument électrochirurgical selon l'une quelconque des revendications précédentes, **caractérisé en ce qu'**une section isolante (28) est réalisée au niveau d'au moins une des surfaces de coagulation, de façon à éviter tout contact électrique direct entre les surfaces de coagulation (22a, 23a).

14. Instrument électrochirurgical selon l'une quelconque des revendications précédentes, notamment selon la revendication 13, **caractérisé en ce que** la section isolante (28) est réalisée à partir de plusieurs sections partielles (28a, 28a', 28b, 28b').

15. Instrument électrochirurgical selon l'une quelconque des revendications précédentes, notamment selon la revendication 13 ou 14, **caractérisé en ce que** la section isolante (28) est réalisée de façon structurée.

16. Instrument électrochirurgical selon l'une quelconque des revendications précédentes, notamment selon l'une quelconque des revendications 13 à 15, **caractérisé en ce que** la section isolante (28) est réalisée en céramique ou en diamant.

17. Instrument électrochirurgical selon l'une quelconque des revendications précédentes, notamment selon l'une quelconque des revendications 13 à 16, **caractérisé en ce que** la section isolante (28) prend la forme du ou des profils de surface (27, 27') respectifs contribuant à l'effet de serrage.

18. Instrument électrochirurgical selon l'une quelconque des revendications précédentes, **caractérisé en ce que** l'instrument prend la forme d'un instrument laparoscopique.
